# EUROPEAN PATENT APPLICATION

(11) **EP 3 939 987 A1**
(43) Date of publication of application: **19.01.2022**
(21) Application number: 19918597.6
(22) Date of filing: 08.10.2019
(51) Int. Cl.: C07H 21/00, A61K 31/7088, A61P 29/00

(54) **OLIGONUCLEOTIDE HAVING ANTI-INFLAMMATORY ACTIVITY**

(30) Priority: 13.03.2019 KR 20190028575; 23.09.2019 KR 20190116657
(71) Applicant: Park, Donghwi, Daegu 41186 (KR)
(72) Inventor: HAN, Seung Woo, Daegu 42101 (KR); CHO, Hyun Jung, Daegu 41482 (KR); PARK, Donghwi, Daegu 41186 (KR)
(74) Representative: HGF
(86) International application number: PCT/KR2019/013184
(87) International publication number: WO 2020/184799

(57) **Abstract**

The present invention relates to an oligonucleotide having an anti-inflammatory activity, and a pharmaceutical composition for preventing or treating an inflammatory disease, comprising the same.

## Description

### Technical Field

The present invention relates to a synthetic oligonucleotide that has an anti-inflammatory activity and can alleviate the cytotoxicity caused by inflammatory stimulation, and a pharmaceutical composition for preventing or treating an inflammatory disease, comprising the same.

### Background Art

Inflammation is an initial defense mechanism of human body against infection or tissue damage, and it is one of the biological defense mechanisms that intend to localize the effect by removing harmful factors and restoring the damaged site to normal when a living body tissue is stimulated from the outside. Various mediators regulate the inflammatory response during a series of processes from initiation of inflammation to resolution of inflammation. In general, the initial tissue damage caused by acute inflammation can be normalized through a physiological defense called resolution. However, if the inflammation continues, tissue damage and loss of functions of cells constituting the tissue are caused.

The causes of inflammation include physical factors such as trauma, frostbite, burns, radiation; chemical factors such as chemicals, ex. acids; and immunological factors such as antibody response. In addition, Inflammation is caused by blood vessel or hormonal imbalance. Cells damaged by external stimuli secrete various biological mediators such as pro-inflammatory cytokines and chemokines, interleukines, and interferons, resulting in vasodilation and increased permeability. As a result, immune cells such as antibodies, complement, plasma, and phagocytes flock to the site of inflammation.

When the inflammatory response, which is an initial defense mechanism of the human body, becomes excessive, the cells not only in the lesion tissue but also in the normal tissue are also destroyed. As a result, when a large amount of purine-based nucleosides such as adenosine and guanosine in the form of adenosine triphosphate (ATP), guanosine triphosphate (GTP), deoxyribonucleic acid (DNA), and ribonucleic acid (RNA) are released in cells, they are decomposed into adenosine and guanosine, respectively, by degrading enzymes such as nucleotidase, CD39, and CD73, which are abundantly present in inflamed immune cells, and they act directly as negative feedback on the inflamed immune cells to regulate the excessive inflammatory response. However, in the case of a severe inflammatory response, if the feedback process of adenosine and guanosine does not work properly, side effects such as pain and septic shock continue due to the excessive inflammatory response.

A drug that removes an inflammatory source and reduces biological responses and symptoms in order to resolve such inflammation is referred to as an anti-inflammatory agent. Substances that have been used for anti-inflammatory purposes so far include non-steroids such as ibuprofen and indomethacin, and steroids such as dexamethasone. The substances exhibit rapid anti-inflammatory action, but symptoms worsen when the use of the substances is discontinued. In particular, steroids cannot be used for a long period of time because they have several problems such as liver and kidney dysfunction, impaired blood sugar, diabetes, hyperlipidemia, and decreased immunity. Accordingly, there is a need to develop substances that can replace the existing anti-inflammatory agents.

Previously, there have been studies confirming that purine-based nucleosides such as adenosine and guanosine exhibit anti-inflammatory effects by participating in a negative feedback response that regulates excessive inflammatory responses when administered to the human body. However, when adenosine and guanosine are directly administered, adenosine also acts on cardiac cells, thereby causing side effects such as cardiac arrest and bradycardia. In addition, even if a small amount of adenosine is administered in combination with guanosine, guanosine promotes the action of adenosine and the side effects are further increased. Because of the above problem, direct administration of adenosine and guanosine is dangerous.

(Patent Document 1) Korean Patent Application Publication No. 10-2018-0021856

### Detailed Description of Invention

### Technical Problem

Under this background, the present inventors found that, when adenosine and guanosine are administered in the form of an oligonucleotide in which adenosine and guanosine are bound to each other, the oligonucleotide are locally degraded into adenosine and guanosine only in a tissue having many inflammatory response-activated immune cells that releases an oligonucleotide degrading enzyme such as nucleotidase, so it can be safely used without systemic side effects. In addition, when the ratio of adenosine and guanosine is between 1:3 and 3:1, a synthetic oligonucleotide exhibits an anti-inflammatory activity without major side effects. Based on the above, the present inventors completed the present invention.

Therefore, an object of the present invention is to provide an oligonucleotide that has an anti-inflammatory activity and may alleviate the cytotoxicity caused by inflammatory stimulation.

Another object of the present invention is to provide a pharmaceutical composition for preventing or treating an inflammatory disease, comprising the oligonucleotide as an active ingredient.

However, the problems to be solved by the present invention are not limited to the problems mentioned above, and other problems that are not mentioned will be clearly understood by those of ordinary skill in the art from the following description.

### Solution to Problem

According to one embodiment of the present invention, there is provided an oligonucleotide having an anti-inflammatory activity, consisting of adenosine and guanosine, wherein the ratio of adenosine and guanosine is between 1:3 and 3:1.

According to one aspect, the length of oligonucleotide may be 10 to 78 bp.

According to one aspect, the oligonucleotide may consist of a nucleotide sequence selected from the group consisting of SEQ ID NO: 2 to SEQ ID NO: 4 and SEQ ID NO: 7 to SEQ ID NO: 14.

According to one aspect, the oligonucleotide may alleviate the cytotoxicity caused by LPS.

According to one aspect, the oligonucleotide may inhibit the production of nitric oxide (NO).

According to one aspect, the oligonucleotide may inhibit the production of an inflammatory cytokine.

According to another embodiment of the present invention, there is provided a pharmaceutical composition for preventing or treating an inflammatory disease, comprising the oligonucleotide having an anti-inflammatory activity as an active ingredient.

According to one aspect, the inflammatory disease may be a brain inflammatory disease or osteoarthritis.

According to one aspect, the brain inflammatory disease may include stroke, Huntington's disease, neuromyelitis optica spectrum disorder, motor neuron disease, Parkinson's disease, Alzheimer's disease, and multiple sclerosis.

### Effects of Invention

The oligonucleotide of the present invention not only exhibits an excellent anti-inflammatory effect, but also can alleviate the cytotoxicity caused by LPS, and it can be locally applied to inflammatory cells in which substances that degrade the oligonucleotide into respective adenosine and guanosine are abundantly present to minimize side effects.

Therefore, the oligonucleotide of the present invention may be used as an anti-inflammatory substance replacing steroids, and may be usefully used for preventing or treating an inflammatory disease such as a brain inflammatory disease and osteoarthritis.

It should be understood that the effects of the present invention are not limited to the above-described effects, and include all effects that can be inferred from the configuration of the invention described in the detailed description or claims of the present invention.

### Brief Description of Drawings

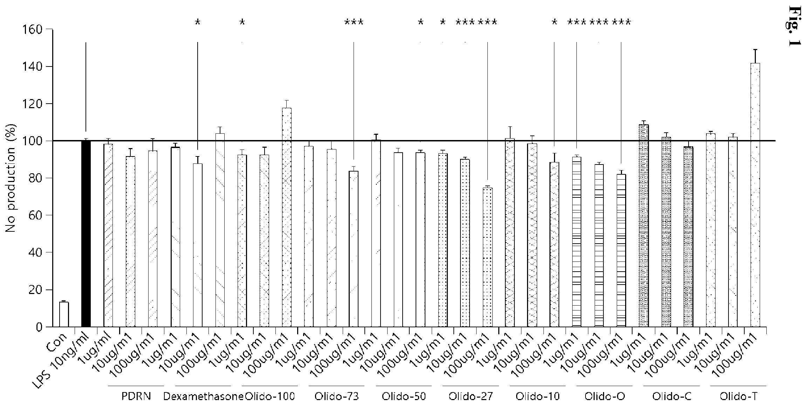

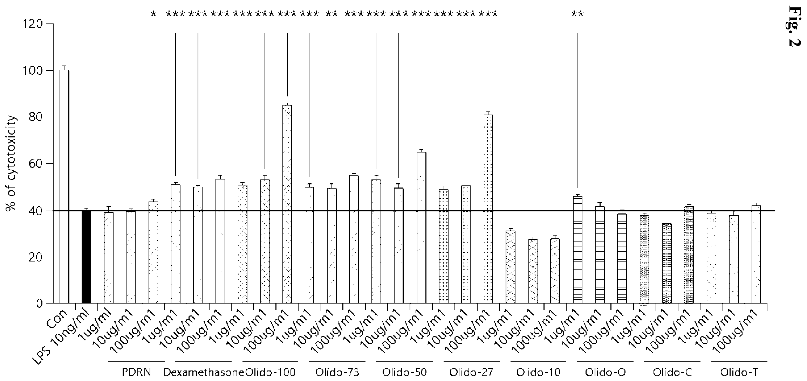

Fig. 1 illustrates a result obtained by confirming the degree of nitric oxide (NO) production by treating RAW cells (mouse macrophage cell line), in which an inflammatory response was induced by LPS, with the oligonucleotides having different ratios of adenosine and guanosine.
Fig. 2 illustrates a result obtained by confirming the degree of cytotoxicity by MTT analysis by treating RAW cells (mouse macrophage cell line), in which an inflammatory response was induced by LPS, with the oligonucleotides having different ratios of adenosine and guanosine.
   In graphs of Figs. 1 and 2, those described in x-axis indicate the following: con (RAW cell), LPS (10 ng/mL 24 hr treatment), PDRN (LPS + PDRN), Dexamethasone (LPS + dexamethasone), Oligo 100 (adenosine 100%), Oligo 73 (adenosine 73.3% + guanosine 26.7%), Oligo 50 (adenosine 50% + guanosine 50%), Oligo 27 (adenosine 26.7% + guanosine 73.3%), Oligo 10 (adenosine 10% + guanosine 90%), Oligo 0 (guanosine 100%), Oligo C (cytidine 100%), and Oligo T (thymidine 100%).

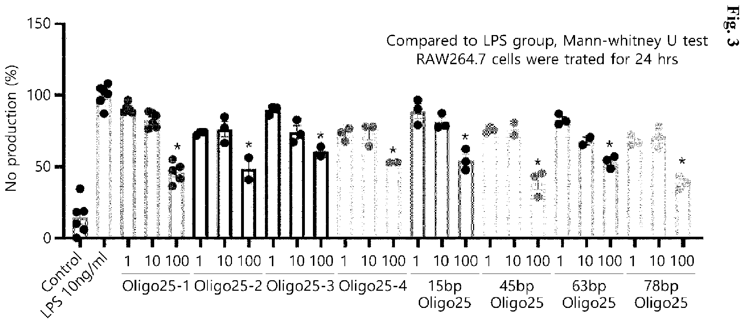

Fig. 3 illustrates a result obtained by confirming the amount of nitric oxide (NO) production by treating RAW cells (mouse macrophage cell line), in which an inflammatory response was induced by LPS, with the oligonucleotides that have the ratio of adenosine and guanosine of 1:3 and have different sequences or lengths.

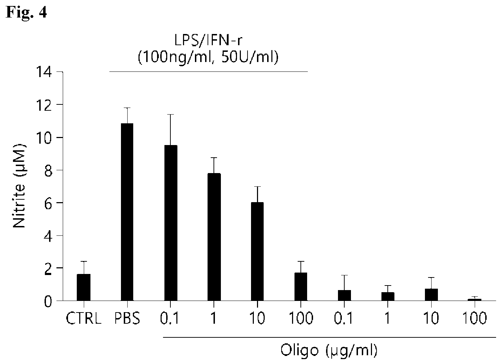

Fig. 4 illustrates a result obtained by confirming the amount of nitric oxide (NO) production by treating a cell line of microglia, in which an inflammatory response was induced by LPS, with the oligonucleotide of SEQ ID NO: 11.

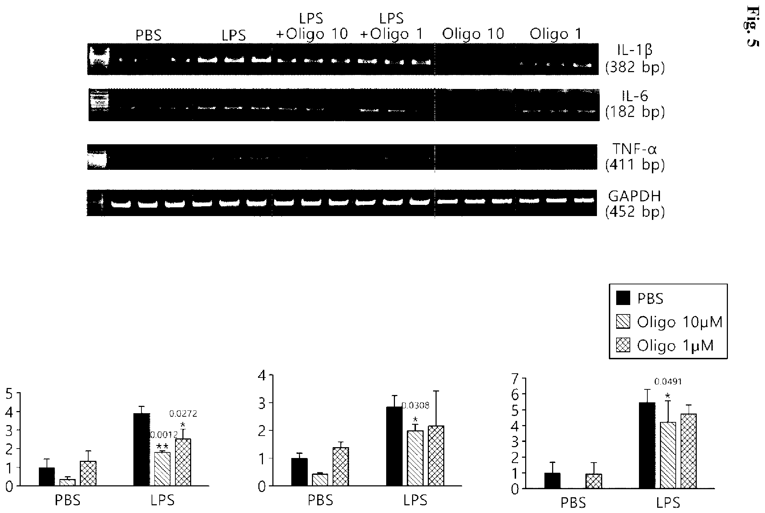

Fig. 5 illustrates a result obtained by confirming the expression amount of pro-inflammatory cytokines IL-1β, IL-6, and TNF-α by treating a cell line of microglia, in which an inflammatory response was induced by LPS, with the oligonucleotide of SEQ ID NO: 11.

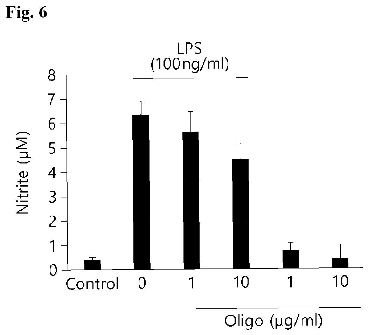

Fig. 6 illustrates a result obtained by confirming the amount of nitric oxide (NO) production by treating mouse astrocytes, in which an inflammatory response was induced by LPS, with the oligonucleotide of SEQ ID NO: 11.

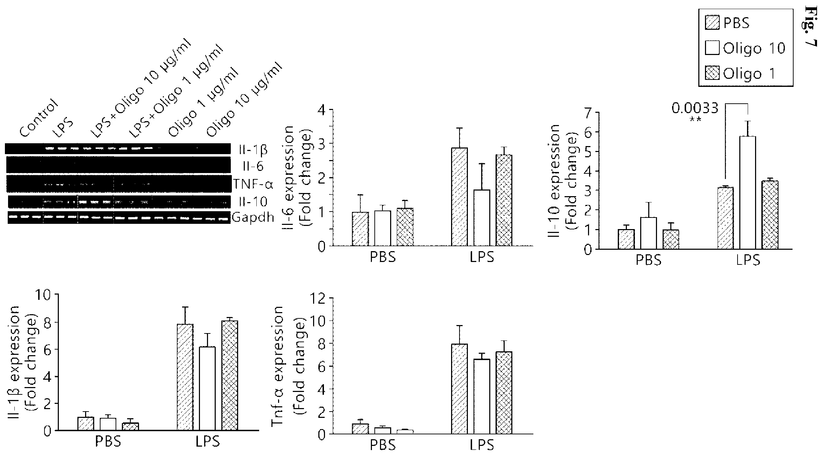

Fig. 7 illustrates a result obtained by confirming the amount of cytokine production by treating mouse astrocytes, in which an inflammatory response was induced by LPS, with the oligonucleotide of SEQ ID NO: 11.

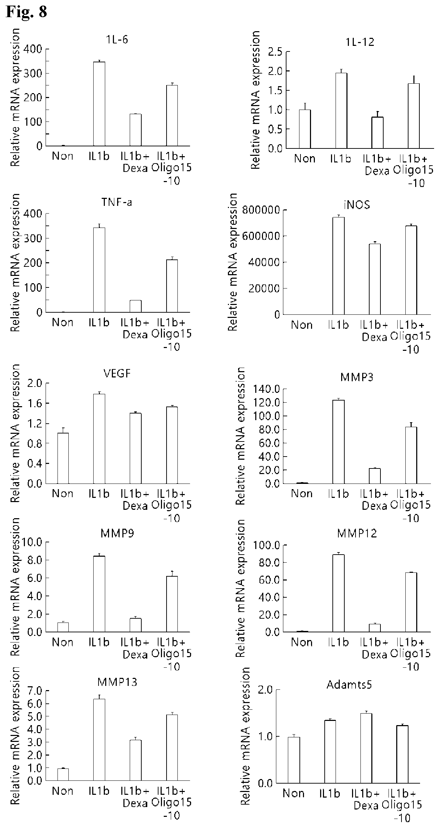

Fig. 8 illustrates a result obtained by confirming the expression amount of cytokines after sequentially treating mouse chondrocytes with the oligonucleotide of SEQ ID NO: 11 and IL-1β.

### Best Mode for Carrying out the Invention

Hereinafter, embodiments will be described in detail with reference to the accompanying drawings. However, since various changes may be made to the embodiments, the scope of the right of the patent application is not limited or restricted by these embodiments. It should be understood that all modifications, equivalents and substitutes for the embodiments are included in the scope of the right.

Terms used in the embodiments are used for the purpose of description only, and should not be construed as limiting. The singular expression includes the plural expression unless the context clearly dictates otherwise. In the present specification, it should be understood that the terms such as "comprise" or "have" are intended to designate that a feature, number, step, operation, component, part, or combination thereof described in the specification exists, but they do not preclude the possibility of the presence or addition of one or more other features, numbers, steps, operations, components, parts, or combinations thereof.

Unless defined otherwise, all terms used herein, including technical or scientific terms, have the same meaning as commonly understood by those of ordinary skill in the art to which the embodiment belongs. Terms such as those defined in a commonly used dictionary should be interpreted as having a meaning consistent with the meaning in the context of the related art, and should not be interpreted as an ideal or excessively formal meaning unless clearly defined in the present application.

In addition, in the description with reference to the accompanying drawings, the same reference numerals are assigned to the same components regardless of figure numerals, and the overlapping description thereof will be omitted. In the description of the embodiments, if it is determined that a detailed description of a related known technology may unnecessarily obscure the gist of the embodiment, the detailed description thereof will be omitted.

The present inventors confirmed the anti-inflammatory effect by synthesizing the oligonucleotides having various ratios of adenosine and guanosine in order to solve the side effects that occur when simply mixing adenosine and guanosine. As a result, it was found that the oligonucleotide having the ratio of adenosine and guanosine in the range between 1:3 and 3:1 exhibits the effect of alleviating the cytotoxicity along with an anti-inflammatory activity, and in particular, the anti-inflammatory activity is maximal at a ratio of about 1:3 (26.7:73.3). Based on the above, the present inventors completed the present invention.

Therefore, according to one embodiment of the present invention, there is provided an oligonucleotide having an anti-inflammatory activity, consisting of adenosine and guanosine, wherein the ratio of adenosine and guanosine is between 1:3 and 3:1. The ratio of adenosine and guanosine may be in the range of, for example, 1:3, 2:3, 1:1, 3:2, 3:1, but is not limited thereto.

When the ratio of adenosine and guanosine is out of the range, it does not exhibit an anti-inflammatory activity or it has the reduced effect of alleviating the cytotoxicity caused by LPS (see Examples 2 and 3). In addition, the ratio of adenosine and guanosine is most preferably 1:3 in terms of satisfying both anti-inflammatory activity and the effect of alleviating the cytotoxicity.

On the other hand, the length of oligonucleotide may be 10 to 78 bp (base pair), preferably 15 to 78 bp, but is not limited thereto. If the length of oligonucleotide is more than 78 bp, the accuracy of synthesis may be reduced. Conversely, if the length of oligonucleotide is less than 10 bp, it is undesirable because it is more easily degraded into adenosine and guanosine in the human body, resulting in increasing the possibility of causing side effects such as cardiac arrest, bradycardia, and the like.

The oligonucleotide of the present invention may consist of a nucleotide sequence selected from the group consisting of SEQ ID NO: 2 to SEQ ID NO: 4 and SEQ ID NO: 7 to SEQ ID NO: 14. However, as confirmed in the Examples below, when the ratio of adenosine and guanosine is in the range between 1:3 and 3:1, the oligonucleotide may exhibit an anti-inflammatory activity regardless of the sequence.

The oligonucleotide of the present invention alleviates the cytotoxicity caused by LPS, inhibits the production of nitric oxide (NO), and inhibits the production of inflammatory cytokines such as IL-1β, IL-6, and TNF-α, thereby exhibiting an excellent anti-inflammatory effect.

According to another embodiment of the present invention, there is provided a pharmaceutical composition for preventing or treating an inflammatory disease, comprising the oligonucleotide having an anti-inflammatory activity as an active ingredient.

The inflammatory disease is a generic term for diseases whose main lesion is inflammation, and may be preferably atopy, psoriasis, dermatitis, allergy, arthritis, rhinitis, otitis media, sore throat, tonsillitis, cystitis, nephritis, pelvic inflammatory disease, Crohn's disease, ulcerative colitis, ankylosing spondylitis, systemic lupus erythematodes (SLE), asthma, edema, delayed allergy (type IV allergy), transplant rejection, graft versus host disease, autoimmune encephalomyelitis, multiple sclerosis, inflammatory bowel disease, cystic fibrosis, diabetic retinopathy, ischemic reperfusion injury, vascular restenosis, glomerulitis, or gastrointestinal allergy, but is not limited thereto. Here, the arthritis refers to a disease caused by inflammatory changes in the joint due to various causes such as bacteria, trauma, autoimmune disease, and the like, and is preferably a brain inflammatory disease or osteoarthritis.

The brain inflammatory disease may include stroke, Huntington's disease, neuromyelitis optica spectrum disorder (NMOSD), motor neuron disease, Parkinson's disease, Alzheimer's disease, and multiple sclerosis, but is not limited thereto.

As used herein, the term "preventing" refers to any action that inhibits or delays the onset of an inflammatory disease by administering the pharmaceutical composition of the present invention.

As used herein, the term "treating" may be construed to include any action to improve or benefit symptoms of an inflammatory disease by administering the pharmaceutical composition of the present invention, but is not particularly limited thereto.

The pharmaceutical composition of the present invention may further comprise appropriate carriers, excipients, and diluents commonly used in the preparation of the pharmaceutical composition, and the carrier may be a non-natural carrier.

The carriers, excipients, and diluents may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

On the other hand, the pharmaceutical composition of the present invention may be formulated in the form of oral formulation such as powders, granules, tablets, capsules, suspensions, emulsions, syrups, and aerosols; external preparations; suppositories; and sterile injection solutions according to conventional methods. When it is formulated, it is prepared using diluents or excipients such as fillers, extenders, binders, wetting agents, disintegrants, and surfactants that are usually used.

Solid preparations for oral administration include tablets, pills, powders, granules, capsules, and the like, and these solid preparations are prepared by mixing at least one excipient, for example, starch, calcium carbonate, sucrose or lactose, gelatin, and the like into the stem cell extract. In addition to simple excipients, lubricants such as magnesium stearate and talc may be also used. Liquid preparations for oral administration include suspensions, internal solutions, emulsions, syrups, and the like, and may comprise various excipients, for example, wetting agents, sweeteners, flavoring agents, and preservatives, in addition to water and liquid paraffin, which are simple diluents commonly used.

Preparations for parenteral administration include sterile aqueous solutions, non-aqueous solutions, suspensions, emulsions, freeze-dried preparations, and suppositories. As a non-aqueous solvent and suspending agent, propylene glycol, polyethylene glycol, vegetable oils such as olive oil, and injectable esters such as ethyl oleate can be used. As a base of the suppository, witepsol, macrogol, tween 61, cacao butter, laurin butter, glycerogelatin, and the like can be used.

The pharmaceutical composition of the present invention may be administered in a pharmaceutically effective amount. As used herein, the term "pharmaceutically effective amount" refers to an amount that is sufficient to treat a disease with a reasonable benefit/risk ratio applicable to medical treatment. The effective dose level may be determined according to factors including the type, severity, age, and sex of the subject, the activity of the drug, the sensitivity to the drug, the administration time, the route of administration, and the excretion rate, the duration of treatment, drugs used concurrently, and other factors well known in the medical field.

The pharmaceutical composition of the present invention may be administered as an individual therapeutic agent or may be administered in combination with other therapeutic agents, and may be administered sequentially or simultaneously with conventional therapeutic agents. In addition, it may be administered in a single dose or multiple doses. Taking all of the above factors into consideration, it is important to administer an amount that can obtain the maximum effect with a minimum amount without side effects.

On the other hand, as used herein, the term "administration" refers to introducing the pharmaceutical composition of the present invention to a subject by any appropriate method, and the route of administration may be through various routes as long as it can reach a target tissue.

Examples of the route of administration may include oral, parenteral, subcutaneous, intraperitoneal, intrapulmonary, and intranasal administration; and parenteral injection includes intramuscular, intraarticular, intraspinal, intraspinal epidural space, intravenous, intraarterial, intraperitoneal, or subcutaneous administration. For parenteral administration of the composition, it may be preferable to prepare a unit dosage form by mixing pharmaceutically acceptable carriers, i.e., those that are non-toxic to the receptor at the concentration and dosage used, and that are miscible with other preparation ingredients under the desired purity.

Hereinafter, the present invention will be described in more detail through the examples. The following examples are described for the purpose of illustrating the present invention, but the scope of the present invention is not limited thereto.

### Example 1: Synthesis of oligonucleotide

The oligonucleotides having a length of 30 bp were synthesized with the ratio of adenosine and guanosine of 100:0, 26.7:73.3, 50:50, 73.3:26.7, 10:90, and 0:100, respectively. The oligonucleotides were synthesized by a method of synthesizing from the 3' end to the 5' end through a 4-step cycle of 1) Deblocking, 2) Coupling, 3) Capping, and 4) Oxidation in an exclusive synthesizer (Mermade 192 (Bioautomation, USA)). Thereafter, the incompletely synthesized oligonucleotides were removed using high affinity purification (HAP).

The specific sequences of the synthesized oligonucleotides are as follows.

**[Table 1]**

| SEQ ID NO | Ratio of Adenosine : Guanosine | Sequence |
|---|---|---|
| 1 | 100:0 | AAA AAA AAA AAA AAA AAA AAA AAA AAA AAA |
| 2 | 26.7:73.3 | AAA GAA AGA AAG AAA GAA AGA AAG AAA GAG |
| 3 | 50:50 | AGA GAG AGA GAG AGA GAG AGA GAG AGA GAG |
| 4 | 73.3:26.7 | AGG GAG GGA GGG AGG GAG GGA GGG AGG GAG |
| 5 | 10:90 | AGG GAG GGA GGG GGG GGG GGG GGG GGG GGG |
| 6 | 0:100 | GGG GGG GGG GGG GGG GGG GGG GGG GGG GGG |

### Example 2: Inhibitory effect of nitric oxide (NO) production induced by LPS treatment

The RAW cells (Sigma-Aldrich) were treated with LPS (lipopolysaccharide), an inflammatory response-inducing substance, at a concentration of 10 ng/mL, and 2 hours later, treated with the oligonucleotide prepared in Example 1 above at a concentration of 1 µg/ml, 10 µg/ml,and 100 µg/ml, and the amount of nitric oxide (NO) produced was compared. The inhibitory effect of NO production was evaluated as the degree of inhibiting NO production by comparing the amount of NO produced in the untreated RAW cells (con) with the amount of NO produced in the RAW cells (con) that were treated with LPS.

As a result, as shown in Fig. 1, it was found that the oligonucleotide (Oligo 100) comprising adenosine only rather worsened the inflammation, and although not shown in the graph, the inflammation was also worsened in the oligonucleotide of adenosine 90% + guanosine 10%. An anti-inflammatory activity started to be shown from the oligonucleotide (Oligo 73) of adenosine 73.3% + guanosine 26.7%; the effect was maximal in the oligonucleotide (Oligo 27) of adenosine 26.7% + guanosine 73.3%; and the effect was shown even in the oligonucleotide (Oligo 10) of adenosine 10% + guanosine 90% and the oligonucleotide (Oligo 0) comprising guanosine only.

That is, it was found that an anti-inflammatory activity was shown only when guanosine was included in the oligonucleotide in a ratio of at least 26.7% or more, and when guanosine was included less than the ratio, the inflammation caused by LPS in macrophages was rather worsened.

### Example 3: Effect of alleviating cytotoxicity caused by LPS treatment

The RAW cells (Sigma-Aldrich) were treated with LPS (lipopolysaccharide) at a concentration of 10 ng/mL to induce an inflammatory response, and then treated with the oligonucleotide prepared in Example 1 above at a concentration of 1 µg/ml, 10 µg/ml, and 100 µg/ml, and cultured for 24 hours. The cytotoxicity was measured by MTT analysis.

As a result, as shown in Fig. 2, when treated with the oligonucleotide (Oligo 10) of adenosine 10% + guanosine 90%, the cytotoxicity caused by LPS was significantly alleviated as compared with the case of treatment with dexamethasone, a steroid. It was confirmed that the effect of alleviating the cytotoxicity was shown up to the oligonucleotide (Oligo 27) of adenosine 26.7% + guanosine 73.3%, but when the ratio of adenosine was higher than that, the effect of alleviating the cytotoxicity was lowered.

When considering the results of Examples 2 and 3 above collectively, as shown in Table 2, the oligonucleotide capable of exhibiting an anti-inflammatory activity while alleviating the cytotoxicity caused by inflammatory stimulation corresponds to the oligonucleotide in which the ratio of adenosine and guanosine is between 1:3 and 3:1.

**[Table 2]**

| | | | | | | |
|---|---|---|---|---|---|---|
| Adenosine | 100% | 73.30% | 50.00% | 26.70% | 10% | 0% |
| Guanosine | 0% | 26.70% | 50.00% | 73.30% | 90% | 100% |
| Ratio (A:G) | 100:0 | 1:3 | 1:1 | 3:1 | 1:9 | 0:100 |
| Anti-inflammatory activity | X | O | O | O | O | O |
| Effect of alleviating cytotoxicity caused by LPS | X | O | O | O | X | X |

### Example 4: Comparison of anti-inflammatory action according to difference in sequence and length of oligonucleotide

In order to confirm whether the anti-inflammatory effect is different depending on the sequence or length of the oligonucleotide, the oligonucleotide in which the ratio of adenosine and guanosine is 1:3 was additionally prepared. The oligonucleotides were prepared using the method described in Example 1 above, and the specific sequences of the prepared nucleotides are as follows.

**[Table 3]**

| SEQ ID NO | Name | Sequence |
|---|---|---|
| 7 | Oligo25-1 | AGG GAG GGA GGG AGG GAG GGA GGG AGG GAG |
| 8 | Oligo25-2 | GGG GAG GAG GGA GGA GGG GGA GAG AGG GGA |
| 9 | Oligo25-3 | AGG GGG GGG GAG GGA GAG GGA AGA GGG GGA |
| 10 | Oligo25-4 | GGG AGA GAG GAG AGG GAG GGA GGA GGG GGG |
| 11 | 15bp Oligo25 | AGG GAG GGA GGG AGG |
| 12 | 45bp Oligo25 | AGG GAG GGA GGG AGG GAG GGA GGG AGG GAG GGA GGG AGG GAG |
| 13 | 63bp Oligo25 | AGG GAG GGA GGG AGG GAG GGA GGG AGG GAG |
| | | GGA GGG AGG GAG GGG AGG GAG GGA GGG AGG |
| 14 | 78bp Oligo25 | AGG GAG GGA GGG AGG GAG GGA GGG AGG GAG GGA GGG AGG GAG GGG AGG GAG GGA GGG AGG GAG GGA GGG AGG GAG |

Next, the RAW cells (Sigma-Aldrich) were treated with LPS (lipopolysaccharide), an inflammatory response-inducing substance, at a concentration of 10 ng/mL, and 2 hours later, treated with the oligonucleotides of SEQ ID NOs: 7 to 14 above at a concentration of 1 µg/ml, 10 µg/ml, and 100 µg/ml, and the amount of nitric oxide (NO) produced was compared. As a result, as shown in Fig. 3, it was confirmed that the amount of NO produced was reduced in proportion to the concentration in the cells treated with the oligonucleotide as compared with the control without any treatment. These results suggest that the anti-inflammatory activity of the oligonucleotide can exhibit a similar anti-inflammatory effect regardless of the sequence or length if the ratio of adenosine and guanosine is in a specific range.

### Example 5: Anti-inflammatory effect in microglia

In order to confirm whether the same anti-inflammatory effect is also exhibited in the microglia, the BV2 cells, a mouse microglia cell line, were treated with LPS (100 ng/ml) + interferon-gamma (50 unit/ml) for inflammatory stimulation, and allowed to stand for 2 hours, and then treated with the oligonucleotide (15 bp Oligo25, SEQ ID NO: 11) to confirm the amount of NO produced and the expression amount of pro-inflammatory cytokines.

The expression amount of cytokines was confirmed by measuring the expression level of cytokine genes in the BV2 cells using conventional PCR analysis, and the specific method is as follows. Total ribonucleic acid (RNA) was extracted from the treated cells using a TRIZOL reagent (Invitrogen, Carlsbad, CA, United States). Reverse transcription (RT) was carried out using Superscript II reverse transcriptase (Invitrogen) and oligo (dT) primers. PCR amplification was carried out in a C1000 Touch Thermal Cycler (Bio-Rad, Richmond, CA, United States) at an annealing temperature of 55-60°C in 25-32 cycles using specific primer sets. The PCR product having ethidium bromide was electrophoresed on a 1% agarose gel and irradiated with ultraviolet rays to observe bands.

As a result, as shown in Figs. 4 and 5, NO production was reduced in proportion to the treatment concentration of the oligonucleotide (Fig. 4), and the expression level of the genes of the pro-inflammatory cytokines IL-1β, IL-6, and TNF-α increased by LPS treatment was significantly reduced (Fig. 5).

### Example 6: Anti-inflammatory effect in astrocyte

In order to confirm whether the same anti-inflammatory effect is also exhibited in the astrocyte cells, the mouse primary astrocytes (made by culturing astrocytes derived from the brain of neonatal mice 2-3 days after birth) were prepared. The prepared astrocytes were treated with LPS (100 ng/ml) + interferon-gamma (50 unit/ml) for inflammatory stimulation, and allowed to stand for 2 hours, and then treated with the oligonucleotide (15 bp Oligo25, SEQ ID NO: 11) to confirm the amount of NO produced and the expression amount of cytokines.

The expression amount of cytokines was confirmed by measuring the expression level of the cytokine genes using the conventional PCR analysis described in Example 5 above, and the concentration of cytokine proteins in the cell culture using Western blotting.

As a result, as shown in Figs. 6 and 7, NO production was reduced in proportion to the treatment concentration of the oligonucleotide (Fig. 6); the expression level in the genes of the pro-inflammatory cytokines IL-1β, IL-6, and TNF-α was significantly reduced; and the expression level in the gene of the anti-inflammatory cytokine IL-10 was also significantly increased (Fig. 7).

### Example 7: Anti-inflammatory effect in chondrocyte

In order to confirm whether the same anti-inflammatory effect is also exhibited in the chondrocytes, the mouse primary chondrocytes were treated with the oligonucleotide (15 bp Oligo25, SEQ ID NO: 11) at a concentration of 10 ng/mL and allowed to stand for 2 hours. Then treated with IL-1β, and 6 hours later, the expression amount of cytokines was confirmed by PCR analysis.

The expression amount of cytokines was confirmed by measuring the expression level of cytokine genes using conventional PCR analysis, and the specific method is as follows. Total ribonucleic acid (RNA) was extracted from the treated cells using a TRIZOL reagent (Invitrogen, Carlsbad, CA, United States). Reverse transcription (RT) was carried out using Superscript II reverse transcriptase (Invitrogen) and oligo (dT) primers. Real-time PCR was carried out using One Step SYBR PrimeScript RT-PCR Kit (Takara Bio, Otsu, Shiga, Japan), and then it was detected using ABI Prism 7000 sequence detection system (Applied Biosystems, California, CA, United States). As an internal control, glyceraldehyde-3-phosphate dehydrogenase (GADPH) was used.

As a result, as shown in Fig. 8, the expression level of MMP13, MMP12, MMP9, and MMP3 genes known to be increased in osteoarthritis was reduced, and the expression level of ADAMTS5 gene known to be reduced in osteoarthritis was increased. From the above, it was found that the markers of osteoarthritis were significantly improved.

From the results of Examples 5 to 7 above, it can be seen that the oligonucleotide of the present invention exhibits an excellent anti-inflammatory effect not only in macrophage cell line, but also in microglia, astrocytes, and chondrocytes. The oligonucleotide may be usefully used in the treatment of a brain inflammatory disease and osteoarthritis in which cranial nerve damage is induced due to an inflammatory response.

As described above, although the examples have been described with reference to the limited drawings, those of ordinary skill in the art may apply various technical modifications and variations based on the above. For example, even if the described techniques are performed in an order different from the described method, and/or the described components are associated or combined in a form different from the described method, or replaced or substituted by other components or equivalents, appropriate results can be achieved.

Therefore, other implementations, other embodiments, and equivalents to the patent claims are also within the scope of the following claims.

## Claims

1. An oligonucleotide having an anti-inflammatory activity, consisting of adenosine and guanosine, wherein the ratio of adenosine and guanosine is between 1:3 and 3:1.

2. The oligonucleotide of claim 1, wherein the length of oligonucleotide is 10 to 78 bp.

3. The oligonucleotide of claim 1, wherein the oligonucleotide consists of a nucleotide sequence selected from the group consisting of SEQ ID NO: 2 to SEQ ID NO: 4 and SEQ ID NO: 7 to SEQ ID NO: 14.

4. The oligonucleotide of claim 1, wherein the oligonucleotide alleviates the cytotoxicity caused by LPS.

5. The oligonucleotide of claim 1, wherein the oligonucleotide inhibits the production of nitric oxide (NO).

6. The oligonucleotide of claim 1, wherein the oligonucleotide inhibits the production of an inflammatory cytokine.

7. A pharmaceutical composition for preventing or treating an inflammatory disease, comprising the oligonucleotide having an anti-inflammatory activity of claim 1 as an active ingredient.

8. The pharmaceutical composition of claim 7, wherein the inflammatory disease is a brain inflammatory disease or osteoarthritis.

9. The pharmaceutical composition of claim 8, wherein the brain inflammatory disease includes stroke, Huntington's disease, neuromyelitis optica spectrum disorder, motor neuron disease, Parkinson's disease, Alzheimer's disease, and multiple sclerosis.


Sequence Listing
<110> Missionary Benedictine sisters of Tutzing Daegu priory
<120> Oligonucleotide having anti-inflammatory effect
<130> FPC-2019-0151PCT
<150> KR 10-2019-0028575
<151> 2019-03-13
<150> KR 10-2019-0116657
<151> 2019-09-23
<160> 14
<170> KoPatentIn 3.0
<210> 1
<211> 30
<212> DNA
<213> Artificial Sequence
<220>
<223> Oligo 100
<400> 1
aaaaaaaaaa aaaaaaaaaa aaaaaaaaaa 30
<210> 2
<211> 30
<212> DNA
<213> Artificial Sequence
<220>
<223> Oligo 27
<400> 2
aaagaaagaa agaaagaaag aaagaaagag 30
<210> 3
<211> 30
<212> DNA

<213> Artificial Sequence
<220>
<223> Oligo 50
<400> 3
agagagagag agagagagag agagagagag 30
<210> 4
<211> 30
<212> DNA
<213> Artificial Sequence
<220>
<223> Oligo 73
<400> 4
agggagggag ggagggaggg agggagggag 30
<210> 5
<211> 30
<212> DNA
<213> Artificial Sequence
<220>
<223> Oligo 10
<400> 5
agggagggag gggggggggg gggggggggg 30
<210> 6
<211> 30
<212> DNA
<213> Artificial Sequence
<220>
<223> Oligo 0
<400> 6
gggggggggg gggggggggg gggggggggg 30
<210> 7
<211> 30
<212> DNA
<213> Artificial Sequence
<220>
<223>
<400> 7
agggagggag ggagggaggg agggagggag 30
<210> 8

<211> 30
<212> DNA
<213> Artificial Sequence
<220>
<223> Oligo25-2
<400> 8
ggggaggagg gaggaggggg agagagggga 30
<210> 9
<211> 30
<212> DNA
<213> Artificial Sequence
<220>
<223> Oligo25-3
<400> 9
aggggggggg agggagaggg aagaggggga 30
<210> 10
<211> 30
<212> DNA
<213> Artificial Sequence
<220>
<223> Oligo25-4
<400> 10
gggagagagg agagggaggg aggagggggg 30
<210> 11
<211> 15
<212> DNA
<213> Artificial Sequence
<220>
<223> 15bp Oligo25
<400> 11
agggagggag ggagg 15
<210> 12
<211> 42
<212> DNA
<213> Artificial Sequence
<220>
<223> 45bp Oligo25
<400> 12

agggagggag ggagggaggg agggagggag ggagggaggg ag 42
<210> 13
<211> 60
<212> DNA
<213> Artificial Sequence
<220>
<223> 63bp Oligo25
<400> 13
agggagggag ggagggaggg agggagggag ggagggaggg aggggaggga gggagggagg 60
<210> 14
<211> 75
<212> DNA
<213> Artificial Sequence
<220>
<223> 78bp Oligo25
<400> 14
agggagggag ggagggaggg agggagggag ggagggaggg aggggaggga gggagggagg 60
gagggaggga gggag 75

<110>     Missionary Benedictine sisters of Tutzing Daegu priory

<120>     Oligonucleotide having anti-inflammatory effect

<130>     FPC-2019-0151PCT

<150>     KR 10-2019-0028575
<151>     2019-03-13

<150>     KR 10-2019-0116657
<151>     2019-09-23

<160>     14

<170>     KoPatentIn 3.0

<210>     1
<211>     30
<212>     DNA
<213>     Artificial Sequence

<220>
<223>     Oligo 100


<400>     1
aaaaaaaaaa aaaaaaaaaa aaaaaaaaaa                                        30


<210>     2
<211>     30
<212>     DNA
<213>     Artificial Sequence

<220>
<223>     Oligo 27


<400>     2
aaagaaagaa agaaagaaag aaagaaagag                                        30


<210>     3
<211>     30
<212>     DNA
<213>     Artificial Sequence

<220>
<223>     Oligo 50


<400>     3
agagagagag agagagagag agagagagag                                        30


<210>     4
<211>     30
<212>     DNA
<213>     Artificial Sequence

<220>
<223>     Oligo 73

<400>    4
agggagggag ggagggaggg agggagggag                                        30


<210>    5
<211>    30
<212>    DNA
<213>    Artificial Sequence

<220>
<223>    Oligo 10


<400>    5
agggagggag gggggggggg gggggggggg                                        30


<210>    6
<211>    30
<212>    DNA
<213>    Artificial Sequence

<220>
<223>    Oligo 0


<400>    6
gggggggggg gggggggggg gggggggggg                                        30


<210>    7
<211>    30
<212>    DNA
<213>    Artificial Sequence

<220>
<223>    Oligo25-1


<400>    7
agggagggag ggagggaggg agggagggag                                        30


<210>    8
<211>    30
<212>    DNA
<213>    Artificial Sequence

<220>
<223>    Oligo25-2


<400>    8
ggggaggagg gaggaggggg agagagggga                                        30


<210>    9
<211>    30
<212>    DNA
<213>    Artificial Sequence

<220>

<223>    Oligo25-3

<400>    9
aggggggggg agggagaggg aagaggggga                                        30

<210>    10
<211>    30
<212>    DNA
<213>    Artificial Sequence

<220>
<223>    Oligo25-4

<400>    10
gggagagagg agagggaggg aggagggggg                                       30

<210>    11
<211>    15
<212>    DNA
<213>    Artificial Sequence

<220>
<223>    15bp Oligo25

<400>    11
agggagggag ggagg                                                        15

<210>    12
<211>    42
<212>    DNA
<213>    Artificial Sequence

<220>
<223>    45bp Oligo25

<400>    12
agggagggag ggagggaggg agggagggag ggagggaggg ag                          42

<210>    13
<211>    60
<212>    DNA
<213>    Artificial Sequence

<220>
<223>    63bp Oligo25

<400>    13
agggagggag ggagggaggg agggagggag ggagggaggg aggggaggga gggagggagg       60

                                                                        60

<210>    14
<211>    75

<212>    DNA
<213>    Artificial Sequence

<220>
<223>    78bp Oligo25


<400>    14
agggagggag ggagggaggg agggagggag ggagggaggg aggggaggga gggagggagg    60

gagggaggga gggag                                                     75